# EUROPEAN PATENT SPECIFICATION

(11) **EP 1 116 489 B1**
(45) Date of publication and mention of the grant of the patent: **27.04.2005**
(21) Application number: 99943450.9
(22) Date of filing: 20.09.1999
(51) Int. Cl.: A61K 31/70, A61K 9/00, A61K 9/48, A61K 9/02, A61K 9/28, A61K 9/20

(54) **ORAL DRUG DELIVERY SYSTEM FOR ENHANCING THE BIOAVAILABILITY OF ACTIVATED GLYCYRRHETIN**
SYSTEM ZUR ORALEN WIRKSTOFFABGABE ZUR STEIGERUNG DER BIOVERFÜGBARKEIT VON AKTIVIERTEM GLYCYRRHETIN
SYSTEME D'ADMINISTRATION ORALE DE MEDICAMENT PERMETTANT D'AMELIORER LA BIODISPONIBILITE DE LA GLYCYRRHETINE ACTIVEE

(30) Priority: 21.09.1998 JP 28604098
(43) Date of publication of application: 18.07.2001
(73) Proprietor: AMATO PHARMACEUTICAL PRODUCTS, LTD., Fukuchiyama-shi, Kyoto 620-0932 (JP)
(72) Inventor: TAKADA, Kanji, Shimogyo-ku Kyoto-shi Kyoto 600-8040 (JP)
(74) Representative: Kinzebach, Werner, Dr.
(86) International application number: PCT/JP1999/005153
(87) International publication number: WO 2000/016784

(56) References cited:
- EP-A- 0 531 524
- EP-A- 0 535 704
- WO-A-99/29299
- WO-A1-94/10983
- JP-A- 3 255 037
- JP-A- 6 192 107
- JP-A- 8 253 413
- JP-A- 9 087 169
- JP-A- 10 226 650
- JP-A- 58 501 174
- US-A- 4 894 234
- US-A- 5 719 122
- PATENT ABSTRACTS OF JAPAN vol. 016, no. 054 (C-0909), 12 February 1992 (1992-02-12) & JP 03 255037 A (SANTEN PHARMACEUT CO LTD), 13 November 1991 (1991-11-13)
- PATENT ABSTRACTS OF JAPAN vol. 017, no. 046 (C-1021), 28 January 1993 (1993-01-28) & JP 04 261117 A (SANTEN PHARMACEUT CO LTD), 17 September 1992 (1992-09-17)
- PATENT ABSTRACTS OF JAPAN vol. 010, no. 292 (C-376), 3 October 1986 (1986-10-03) & JP 61 109710 A (TAISHO PHARMACEUT CO LTD), 28 May 1986 (1986-05-28)
- PATENT ABSTRACTS OF JAPAN vol. 007, no. 252 (C-194), 9 November 1983 (1983-11-09) & JP 58 140012 A (KODAMA KK), 19 August 1983 (1983-08-19)
- F. TAKAHASHI: 'Recent Progress in Drug Delivery System' JOURNAL OF MEDICINE, vol. 34, S-1, 01 January 1998, pages 237 - 242, XP002925896
- WILSON CLIVE G. ET AL.: 'Evaluation of a gastro-resistant pulsed release delivery system (Pulsincap) in Humans' DRUG DELIVERY, vol. 4, no. 3, 1997, pages 201 - 206, XP002925897
- S. MASUDA, K. TAKADA: 'The Latest Drug Delivery System to the Large Intestine' PHARM. TECH. JAPAN, vol. 11, no. 11, 1995, pages 37 - 46, XP002925898

## Description

The present invention relates to a drug delivery system for oral administration of glycyrrhizin. More particularly, it relates to such a drug delivery system for enhancing the bioavailability of glycyrrhizin.

Glycyrrhizin is a naturally occurring substance found in licorice root (*Glycyrrhiza glabra L*.) that has long been used as a Chinese medicine. It has a strong sweet taste and is used as a sweetener. In addition, glycyrrhizin is used in the treatment of chronic hepatitis, allergic disorder and other pathological conditions by its intravenous administration.

Chemically glycyrrhizin is a glucuronide of glycyrrhetic acid with two moles of glucuronic acid. Several studies on the pharmacokinetic behavior of glycyrrhizin in human and animals have revealed that glycyrrhizin is scarcely detectable in the blood following oral administration but its hydrolyzed product, glycyrrhetic acid, is detectable in the blood. See, Wang, Z. *et al.,* Biol. Pharm. Bull. 17(10):1390-1403(1994); Yamamura, Y. *et al., ibid.,* 18(2):337-341(1995); and Takeda, S. *et al.,* J. Pharm. Pharmacol. 48:902-905(1996). Accordingly, it has been considered that glycyrrhizin is poorly absorbable from digestive tract as such (active form), and hydrolyzed in the digestive tract into glycyrrhetic acid which is known to have only little therapeutic effect on hepatitis. Since glycyrrhizin is mainly absorbed in this form, its bioavailability is very low.

In order to enhance the bioavailability, rectal administration of glycyrrhizin in the form of a rectal suppository was proposes (JP-A-01294619, JP-A-03002122 JP-A-03123731 and JP-A-04261117). Other attempts to enhance absorbability from digestive tract include providing an enteric oral preparation containing a fatty acid glyceride (JP-A-03255037) and an oral preparation containing a lipid emulsion or lipid complex (JP-A-06192107). With these prior art suppository and oral preparations, however, the blood concentration of glycyrrhizin does not reach a level sufficient to exhibit its therapeutic effect.

EP-A 535 704 discloses a glycyrrhizin suppository comprising glycyrrhic acid or salts thereof and a non-ionic surface active agent in addition to an oleaginous base for suppositories.

US-A 5,637,319 describes oral controlled-release preparations suitable to deliver drugs to different sites of gastro-intestinal tract and suitable to control the release rate of drugs. One embodiment is an oral intestinal pressure-controlled release preparation. When the preparation, such as an ethylcellulose made capsule, is disintegrated by the inner pressure of the large intestine, drug solution or drug suspension contained in the capsule is released.

There exists a need for a method and device for enhancing the bioavailability of active glycyrrhizin in oral route to a therapeutically effective level.

According to the present invention, the above need is met by providing a device for colon-targeted oral delivery of glycyrrhizin comprising a shaped core containing an amount of glycyrrhizin, said shaped core being made of a suppository base that comprises higher fatty acid di- and triglycerides and melts or liquefies at the body temperature, and a coating film of ethylcellulose enclosing said shaped core and having a film thickness whereby when the device is transported through the digestive tract to the colon, the film enclosing the liquefied core ruptures selectively in the colon by the internal pressure generated by the peristalsis of the intestine.

When glycyrrhizin is formulated into such drug delivery systems (DDS), it is released selectively in the colon at such a high concentration that saturates and overwhelms the rate of hydrolysis by the intestinal flora and, therefore, the majority of glycyrrhizin may be absorbed in the active form from the colon to achieve remarkably improved bioavailability.

The drug delivery system according to the present invention basically comprises a core portion containing glycyrrhizin in admixture with a pharmaceutically acceptable carrier and a skin or shell portion enclosing the core portion.

Glycyrrhizin is released and absorbed selectively in the colon upon rupture of the skin or shell portion in the colon .

Preferably, the core portion contains glycyrrhizin in an amount sufficient to compensate for and substantially overwhelm the rate of hydrolysis of glycyrrhizin by the intestinal flora.

The term "glycyrrhizin" as used herein includes free glycyrrhizin and a salt thereof such as sodium, potassium or ammonium salt.

Preferably all or a portion of the carrier consists of a substance that promotes the absorption of glycyrrhizin. Examples of such absorption promoters include a pharmaceutically acceptable organic acid such as citric, malic, maleic, fumaric or tartaric acid; a surfactant such as sodium lauryl sulfate, polyoxyethylene sorbitan, polyoxyethylene hydrogenated castor oil, polyoxyethylene alkyl ether, polyoxyethylene alkylphenyl ether, deoxycholate or ursodeoxycholate; or a chelating agent such as EDTA.

The core portion may be produced by a method similar to those methods well known in the art for preparing oral solid preparations. It has been known in the art that the transit time of unit dosage forms for oral administration through the small intestine after expulsion from the stomach and before arrival at the large intestine (small intestine-transit time) is at around 3-4 hours whereas the time required for expulsion of such dosage forms from the stomach after administration may vary to a large extent. It is also known that a distinct rise in pH in the digestive tracts from around 1-3 in the stomach to around 6-7 in the small intestine. The colon-targeting DDS according to the present invention may be designed on the basis of these known physiological phenomenon. Now the colon-targeting DDS of the present invention will be described in detail.

Since glycyrrhizin must be released in the large intestine at higher concentrations as noted above, unit dosage forms similar to rectal suppositories are well suited as a capsule content for this purpose. The method for preparing such unit dosage forms is well known in the art and comprises the steps of adding glycyrrhizin to a suitable suppository base such as Witepsol™ H15 (higher fatty acid di- and triglycerides available from Dynamit Nobel) while being in molten state to make a suspension, casting the suspension into a mold and then cooling the mold to solidify the suspension. The resulting suppository-like dosage forms are encapsulated by forming a coating film on the surfaces thereof, preferably by the dipping method.

The DDS of the invention are pressure-collapsible, colon-targeted delivery capsules.

This system is also disclosed in U.S. Patent No. 5,637,319 to Takada and Seizai-to-Kikai (Pharmaceutical preparations and machines), January 15, 1998 issue.

This capsule is collapsed in the colon as follows. Ingested diet is flowable in the stomach and small intestine because of abundant presence of water-containing digestive juice whereas the content in the large intestine becomes highly viscous since reabsorption of water and formation of stool take place there. In such a dense environment, the capsule will rupture by the internal pressure associated with the peristalsis of colon to release the drug contained therein.

The capsule is made of ethylcellulose or gelatin lined with ethylcellulose inside. Since the capsule content must be a liquid form when the capsule is collapsed, glycyrrhizin is contained in the capsule as a solution or dispersion in a base for making rectal suppositories which liquify at the body temperature. The rupture time in the colon may be controlled by varying the wall thickness of the ethylcellulose capsule.

Indications of the glycylrrhizin preparation of the present invention include hepatic dysfunction in chronic hepatic disease, various eczema, drug rush, stomatitis, infant strophulus, phlyctena, alopecia areata and the like. The dose of the glycyrrhizin preparation of the present invention may be determined depending on the age, body weight, the type and severity of disease of a particular patient. The daily dose for adult patients (60kg of body weight) with chronic hepatic disease ranges between 10mg and 1,000mg, preferably between 100mg and 800mg as glycyrrhizin. This dose may be administered at once or divided twice or more.

### EXAMPLE

The invention will be described in detail by making reference to the following non-limiting examples.

### Example 1

100mg of glycyrrhizin potassium salt was dispersed in 400mg of molten Witepsol™ H15 (higher fatty acid di- and triglyceride) heated at 50°C. The dispersion was poured into a mold and then cooled well to 6°C to obtain a solid article having supository-like shape. This shaped article was dusted with fine talcum powder and then coated with ethylcellulose film by the dipping method to thereby obtain a intra-colon pressure collapsible, colon-targeted delivery capsule.

### In vivo bioavailability test

### Method:

The test preparation was orally administered with 50ml of water to adult beagle dogs having been fasted overnight for 12 hours. After administration, 2ml of blood samples were collected periodically over 24 hours from the jugular vein and assayed for plasma glycyrrhizin levels using HPLC. Commercial glycylrrhizin tablets (Glycyron™) were used as a control drug. The dose was 100mg as glycyrrhizin.

### Results:

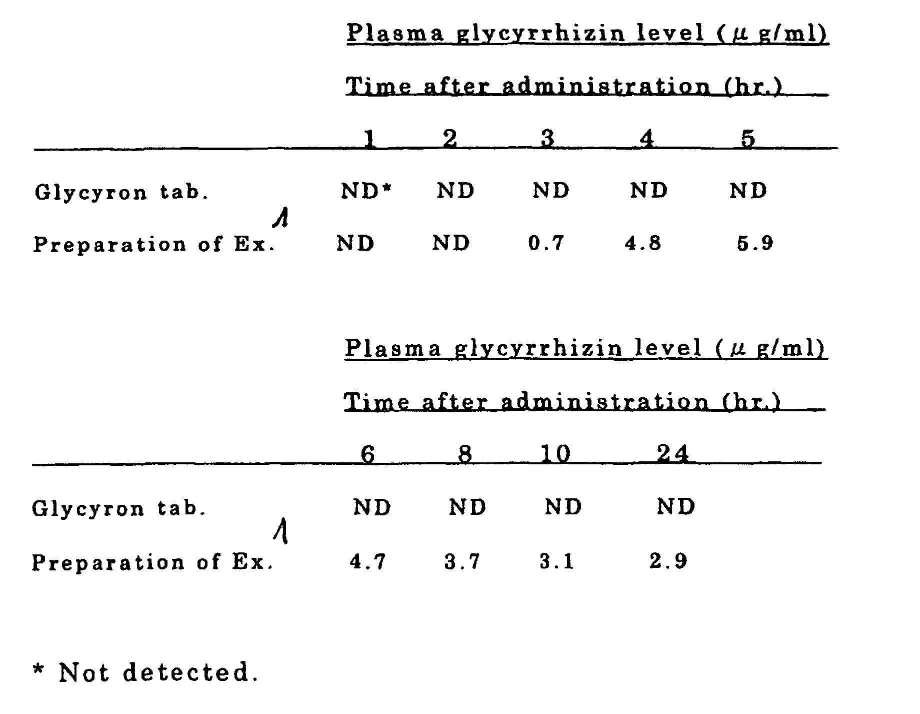

As shown in the above table, glycyrrhizin was not detected in the plasma after oral administration of commercial glycyrrhizin tables. In contrast, when the colon-targeted DDS capsule of Example 1 was orally administered, the plasma glycyrrhizin level began to rise 3 hours after the administration, reached a peak in 4-5 hours and remained at a therapeutically effective level at least up to 24 hours after the administration.

## Claims

1. A device for colon-targeted oral delivery of glycyrrhizin comprising a shaped core containing an amount of glycyrrhizin, said shaped core being made of a suppository base that comprises higher fatty acid di- and triglycerides and melts or liquefies at the body temperature, and a coating film of ethylcellulose enclosing said shaped core and having a film thickness whereby when the device is transported through the digestive tract to the colon, the film enclosing the liquefied core ruptures selectively in the colon by the internal pressure generated by the peristalsis of the intestine.

2. The device according to claim 1, wherein said amount of glycyrrhizin is in excess of the amount needed for compensating for the hydrolysis thereof by the intestinal flora.

3. The device according to claim 1, obtainable by dipping the shaped core in a solution of ethylcellulose.

4. The device according to claim 3, obtainable by dusting the shaped core with a powder to prevent from sticking before dipping.

5. The device according to claim 1, wherein said shaped core further contains an absorption promoter for glycyrrhizin.

6. The device according to claim 4, wherein the powder is talc.

7. The device according to claim 5, wherein absorption promoter is an organic acid, a surfactant, a chelating agent, or a mixture thereof.

8. A process for preparing a colon-targeted oral delivery system of glycyrrhizin comprising:
(a) adding glycyrrhizin to a suppository base that comprises higher fatty acid di- and triglyderide and melts or liquefies at the body temperature while the suppository base is in molten or liquefied state to obtain a suspension;
(b) casting the suspension in a mold;
(c) cooling the mold to obtain a shaped solidified core of the suspension;
(d) enclosing the resultant shaped core with a coating film of ethylcellulose, the coating film having a film thickness whereby when the system is transported through the digestive tract to the colon, the film enclosing the liquefied core ruptures selectively in the colon by the internal pressure generated by the peristalsis of the intestine.

9. The process according to claim 8, wherein the amount of glycyrrhizin in said suspension is in excess of the amount needed for compensating for the hydrolysis of glycyrrhizin by the intestinal flora.

10. The process according to claim 8, wherein said coating film is formed by dipping the shaped core in a solution of ethylcellulose.

11. The process according to claim 8, wherein said suspension further contains an absorption promoter for glycyrrhizin.

12. The process according to claim 8, further comprising dusting the shaped core with powder before (d) to prevent sticking of the shaped core together.

13. The process according to claim 12, wherein the powder is talc.

## Patentansprüche

1. Orale Darreichungsform zur gezielten Abgabe von Glycyrrhizin im Dickdarm, umfassend einen eine Glycyrrhizinmenge enthaltenden Formkern aus einer Suppositoriengrundlage, die höhere Fettsäuredi- und -triglyceride umfasst und bei Körpertemperatur schmilzt oder sich verflüssigt, und einen Überzugsfilm aus Ethylcellulose, der den Formkern einschließt und eine Filmdicke aufweist, so dass beim Transport der Darreichungsform durch den Verdauungstrakt zum Dickdarm der den verflüssigten Kern einschließende Film durch den von der Perestaltik des Darms erzeugten inneren Druck selektiv im Dickdarm aufreißt.

2. Darreichungsform nach Anspruch 1, wobei die Glycyrrhizinmenge größer ist als die zur Kompensation seiner Hydrolyse durch die Darmflora erforderliche Menge.

3. Darreichungsform nach Anspruch 1, dadurch erhältlich, dass man den Formkern in eine Lösung von Ethylcellulose eintaucht.

4. Darreichungsform nach Anspruch 3, dadurch erhältlich, dass man den Formkern vor dem Eintauchen mit einem Pulver bestäubt, um das Aneinanderkleben zu verhindern.

5. Darreichungsform nach Anspruch 1, wobei der Formkern außerdem einen Glycyrrhizin-Absorptionspromotor enthält.

6. Darreichungsform nach Anspruch 4, wobei es sich bei dem Pulver um Talk handelt.

7. Darreichungsform nach Anspruch 5, wobei es sich bei dem Absorptionspromotor um eine organische Säure, ein oberflächenaktives Mittel, einen Komplexbildner oder ein Gemisch davon handelt.

8. Verfahren zur Herstellung einer oralen Darreichungsform zur gezielten Abgabe von Glycyrrhizin im Dickdarm, bei dem man:
(a) Glycyrrhizin zu einer Suppositoriengrundlage im geschmolzenen oder verflüssigten Zustand unter Erhalt einer Suspension gibt, wobei die Suppositoriengrundlagen höhere Fettsäuredi- und triglyceride umfasst und bei Körpertemperatur schmilzt oder sich verflüssigt;
(b) die Suspension in eine Form gießt;
(c) die Form abkühlt, wobei man einen verfestigten Formkern der Suspension erhält;
(d) den erhaltenen Formkern in einen Überzugsfilm aus Ethylcellulose einschließt, wobei der Überzugsfilm eine Filmdicke aufweist, so dass beim Transport des Systems durch den Verdauungstrakt zum Dickdarm der den verflüssigten Kern einschließende Film durch den von der Perestaltik im Darm erzeugten inneren Druck selektiv im Dickdarm aufreißt.

9. Verfahren nach Anspruch 8, wobei die Glycyrrhizinmenge größer ist als die zur Kompensation der Hydrolyse von Glycyrrhizin durch die Darmflora erforderliche Menge.

10. Verfahren nach Anspruch 8, wobei man den Überzugsfilm ausbildet, indem man den Formkern in eine Lösung von Ethylcellulose eintaucht.

11. Verfahren nach Anspruch 8, wobei die Suspension außerdem einen Glycyrrhizin-Absorptionspromotor enthält.

12. Verfahren nach Anspruch 8, bei dem man außerdem den Formkern vor (d) mit einem Pulver bestäubt, um das Aneinanderkleben des Formkerns zu verhindern.

13. Verfahren nach Anspruch 12, wobei es sich bei dem Pulver um Talk handelt.

## Revendications

1. Dispositif pour l'administration orale ciblée vers le côlon de glycyrrhizine, comprenant un coeur moulé contenant une quantité de glycyrrhizine, ledit coeur moulé étant réalisé en une base de suppositoire qui comprend des di- et triglycérides d'acide gras supérieur et qui fond ou se liquéfie à la température du corps, et un film d'enrobage d'éthylcellulose entourant ledit coeur moulé et présentant une épaisseur de film telle que lorsque le dispositif est transporté à travers l'appareil digestif jusqu'au côlon, le film entourant le coeur liquéfié se rompt sélectivement dans le côlon sous l'effet de la pression interne générée par l'activité péristaltique de l'intestin.

2. Dispositif selon la revendication 1, dans lequel ladite quantité de glycyrrhizine est en excès de la quantité nécessaire pour compenser son hydrolyse par la flore intestinale.

3. Dispositif selon la revendication 1, pouvant être obtenu par immersion du coeur moulé dans une solution d'éthylcellulose.

4. Dispositif selon la revendication 3, pouvant être obtenu en saupoudrant le coeur moulé avec une poudre pour l'empêcher de coller avant l'immersion.

5. Dispositif selon la revendication 1, dans lequel ledit coeur moulé contient en outre un promoteur d'absorption de la glycyrrhizine.

6. Dispositif selon la revendication 4, dans lequel la poudre est de talc.

7. Dispositif selon la revendication 5, dans lequel le promoteur d'absorption est un acide organique, un tensioactif, un agent chélatant, ou un de leurs mélanges.

8. Procédé de préparation d'un système d'administration orale de glycyrrhizine ciblée vers le côlon comprenant :
(a) l'ajout de glycyrrhizine à une base de suppositoire qui comprend des di- et triglycérides d'acide gras supérieur et qui fond ou se liquéfie à la température du corps, alors que la base de suppositoire est dans un état fondu ou liquéfié, pour obtenir une suspension ;
(b) la coulée de la suspension dans un moule ;
(c) le refroidissement du moule pour obtenir un coeur moulé solidifié de la suspension ;
(d) l'enrobage du coeur moulé résultant avec un film d'enrobage d'éthylcellulose, le film d'enrobage présentant une épaisseur de film telle que lorsque le système est transporté à travers l'appareil digestif jusqu'au côlon, le film entourant le coeur liquéfié se rompt sélectivement dans le côlon sous l'effet de la pression interne générée par l'activité péristaltique de l'intestin.

9. Procédé selon la revendication 8, suivant lequel la quantité de glycyrrhizine dans ladite suspension est en excès de la quantité nécessaire pour compenser l'hydrolyse de la glycyrrhizine par la flore intestinale.

10. Procédé selon la revendication 8, suivant lequel ledit film d'enrobage est formé par immersion du coeur moulé dans une solution d'éthylcellulose.

11. Procédé selon la revendication 8, suivant lequel ladite suspension contient en outre un promoteur d'absorption de la glycyrrhizine.

12. Procédé selon la revendication 8, comprenant en outre le saupoudrage du coeur moulé avec une poudre avant l'étape (d) pour empêcher le coeur moulé de se coller.

13. Procédé selon la revendication 12, suivant lequel la poudre est de talc.
